# EUROPEAN PATENT SPECIFICATION

(11) **EP 2 473 111 B1**
(45) Date of publication and mention of the grant of the patent: **16.03.2016**
(21) Application number: 10762750.7
(22) Date of filing: 27.08.2010
(51) Int. Cl.: A61B 8/00, G01S 15/89

(54) **ULTRASOUND PROBE WITH LARGE FIELD OF VIEW AND METHOD FOR FABRICATING SUCH ULTRASOUND PROBE**
ULTRASCHALLSONDE MIT BREITEM SICHTFELD UND VERFAHREN ZUR HERSTELLUNG EINER DERARTIGEN ULTRASCHALLSONDE
SONDE À ULTRASONS PRÉSENTANT UN GRAND CHAMP DE VISION ET PROCÉDÉ DE FABRICATION DE CELLE-CI

(30) Priority: 03.09.2009 US 239497 P
(43) Date of publication of application: 11.07.2012
(73) Proprietor: Koninklijke Philips N.V., 5656 AE Eindhoven (NL)
(72) Inventor: ROBINSON, Andrew, L., NL-5656 AE Eindhoven (NL)
(74) Representative: van Velzen, Maaike Mathilde
(86) International application number: PCT/IB2010/053858
(87) International publication number: WO 2011/027270

(56) References cited:
- EP-A1- 0 090 567
- EP-A2- 0 468 506
- WO-A1-03/000137
- US-A- 3 881 164
- US-A- 4 870 867
- US-A- 5 797 845
- US-A1- 2004 168 517

## Description

### FIELD OF THE INVENTION

The invention relates to an ultrasound probe which has a large field of view due to a specific geometric arrangement of ultrasound transducer array elements. Furthermore, the invention relates to a method of fabricating an ultrasound probe.

### BACKGROUND OF THE INVENTION

In medical ultrasound applications, ultrasound transducers are generally used for transmission and reception of ultrasonic or acoustic waves during ultrasound imaging.

The region within the patient's body from which an image can be obtained with sufficiently high image quality and sufficiently low levels of artefacts is referred to as the field of view. A transducer's field of view depends on several factors, among them the transducer array element size, the transducer array aperture, and the transducer array geometry.

Many transducers available on the market comprise a 1D array of ultrasound transducer elements. Each element is a separate, nominally-independent transducer that is used to both transmit ultrasound energy into a body of a patient and receive echoes from structures within the body. Medical ultrasound usually operates in the 1-20 MHz frequency range, necessary to provide imaging detail appropriate to the structures with the human body. Lower and higher frequencies may be used for specific applications.

Each element in a 1D array is typically rectangular, with a narrow extent in one direction (commonly referred to as the azimuth direction), a longer extent in an orthogonal direction (commonly referred to as the elevation direction), and a thickness in the direction orthogonal to the other two directions (commonly referred to as range direction). The size of a transducer element, i.e. its width in the azimuth direction and length in the elevation directions, may determine its acceptance angle at a given frequency. This acceptance angle may be the angle at which an element receives enough energy to contribute constructively to an ultrasound signal. As element width increases, the acceptance angle generally decreases. Furthermore, acceptance angle is also a function of frequency; for a given width the acceptance angle generally decreases with increasing frequency.

While a 1D transducer array usually focuses ultrasonic energy in the elevation direction to a fixed focus region by placing a cylindrical acoustic lens on a face of the transducer array, a region of interest may be scanned in the azimuth direction typically in one of two basic formats: "sector scanning" or "linear scanning".

With sector scanning, the field of view may be a section of a circle with its center in the center of the face of the array. With the sector scan format, the ultrasound beams may be steered radially from the center of the array, and an angle of each successive beam may be different. The delays applied to transmit and receive signals may determine both the steering angle and the focus. Transducers that use a sector scan format are commonly referred to as sector arrays or phased arrays. Such transducers may have elements with widths in the order of λ/2, where λ may be defined as the acoustic wavelength at the frequency in the center of the band in which the transducer operates. A typical phased array provides a field of view with a sector width of 90°.

Alternatively, with a linear scan format, each beam may be perpendicular to the face of the array, and the position of the beam along the array face may be translated laterally to sweep out a rectangular field of view. In this case, the beams may not be steered but are only focused. The delays applied to the transmit and receive signals determine the location of the focus. Transducers that use the linear scan format are commonly referred to as linear arrays, and may have elements with widths in the order of λ. The width of the rectangular field of view is typically the same as the azimuthal length of the transducer.

It may also be possible to build arrays that are curved, and which provide a curved field of view. These transducers typically operate with a linear scan format, i.e. with ultrasound beams that are projected perpendicular to the face of the array. The curvature in the field of view may be provided by translating the ultrasound beams along a curved surface rather than by steering them. Curved transducer arrays operating in a linear scan mode are sometimes referred to as "curved linear arrays" (CLA). Curved linear arrays may provide a larger field of view than flat linear arrays, while avoiding the image quality compromises involved with steering acoustic beams. It may also be possible to have a curved field of view that is larger than the 90° sector typically available from a sector array.

It should be noted that the ultrasound beams used with both flat and curved linear arrays can be steered. However, because linear arrays have wider elements in azimuth than do phased arrays, the ultrasound beam cannot be steered as far as with phased arrays. Thus a flat linear array may have a trapezoidal field of view, using steering to image the angled edges of the field of view.

"Beam forming" may refer to a process of combining signals transmitted and received by the array elements into a sum that represents the ultrasound signal received from a single point in the field of view. In general, for a 1D transducer each transducer element is connected to a separate "channel" in the ultrasound system, wherein a channel may comprise a transmitter, a receiver and delay circuits used to control the timing of transmitted and received signals. The time may be controlled to focus ultrasound energy on transmit and to synthesize a focus for the received signals. A delay applied to each channel may be such that the acoustic signal transmitted by each of the array elements is received at a target at the same time. The delay may be the difference in ultrasound-propagation time, determined for example by the speed of sound and the distance of each element from the target.

Because of a combination of the array geometry, steering and/or focus in azimuth provided by the system beam former, and focus in elevation provided by the fixed lens, 1D arrays typically image a section of a "plane", that is, there is no steering or controlled variable focus of acoustic beams in the elevation direction.

Whereas 1D transducer arrays have elements of ultrasound transducers that span the entire elevation dimension of the array, 2D or matrix arrays may be also divided into elements in the elevation direction. When each element is provided with its own ultrasound beam forming electronics so that the delay applied to its signal can be different from that of its neighbouring element, a possibility of steering, translating; and focusing in the elevation direction as well as in azimuth direction may be obtained. The ability to steer and/or translate the ultrasound beam in the elevation direction may enable imaging a volume of space, rather than just within a plane.

The beam forming electronics provided to each ultrasound transducer element in a matrix array may also be referred to as "microbeamformer". The beam forming function of the entire ultrasound system may be partitioned so that part of it takes place in the microbeamformer and the rest in the over-all controlling system. For example, the transducer elements may comprise a piezoelectric layer arrangement which is contacted by electrodes such that by applying a suitable voltage, the transducer elements can be activated to emit ultrasonic vibrations. The voltage may be applied and controlled using a control circuit associated to the piezoelectric layer arrangement of each of the ultrasound transducer elements. Accordingly, each of the ultrasound transducer elements may be provided with its own "microchannel" comprising a transmitter, receiver and delay circuitry. This may allow signals from contiguous groups of elements to be combined together to drive a single system channel; the microbeam former must provide only a relatively small amount of delay to account for the difference between it and the other elements in its group.

As with 1D arrays, the field of view available from a 2-dimensional matrix array may depend on the array geometry and the scan formats that can be supported by its elements. If the array elements are sufficiently small in both azimuth and elevation direction, the acoustic beam may be steered in both directions. Larger array elements may allow less steering. Array elements need not be square; for example, a matrix array with λ elements in azimuth and λ/2 elements in elevation may be used and may be operated with a linear scan format in azimuth and a sector scan format in elevation.

However, matrix arrays of ultrasound transducers may have limitations concerning their field of view. Field of view limitations may be addressed to a certain extent by increasing the size of an aperture to bring the desired field of view within the steering range of the matrix array. However, there may be limits to the extent that this can be exploited, especially for intracavity ultrasound transducers where increasing transducer size is not an option. It may also be possible to increase steering by decreasing the size of the array elements. However, with matrix arrays this may also decrease the amount of area available for microchannel electronics that must fit in a space associated with each of the transducer elements such that there may be limitations on how small the transducer elements may be made.

In order to provide a larger field of view than is possible with steering a flat (sector) array, curved matrix arrays may be used. In such curved matrix arrays, the ultrasound transducer elements may be arranged on a curved surface. An additional advantage of a curved matrix array may be that a larger field of view may be achieved with fewer, larger transducer elements than the smaller phased array elements required for large steering angles.

However, it has been observed that also curved matrix arrays of ultrasound transducer elements may have limitations with respect to the field of view as well with respect to image quality.

US 2004/0168517 A1 discloses a method for manufacturing crossing/intersecting transducer arrays including first and second ultrasonic transducer arrays which intersect centrally of one another.

EP 0 468 506 A2 discloses an ultrasonic transducer comprising a crossed shaped plate of piezo material, a ground electrode plate disposed on one side of the piezo material, a signal electrode plate disposed on the opposite side of the piezo material, and a plurality of grooves defining a plurality of individual transducer elements, each element having a separate signal electrode on a common side of the piezo material.

EP 0 090 567 A1 discloses an ultrasonic sector-scan probe comprising at least an array which is arranged multidimensionally or in plural linear arrays so that the scanning lines by every group intersect substantially at one point in a window for transmitting and receiving ultrasonic waves.

### SUMMARY OF THE INVENTION

Accordingly, there may be a need for an improved ultrasound transducer matrix array for an ultrasound probe which may overcome some of the field of view and image quality limitations of prior curved matrix arrays, particularly for the case where increasing aperture size or reducing element size for more steering to achieve a larger field of view may not be not an option.

It has been found that an ideal curved matrix transducer array may have a spheroidal shape with a surface curved in two directions. However, current array fabrication techniques may not allow for curvature over such a compound surface curve. While it may be easy to fabricate a curved matrix array which may be curved/bent in one direction, i.e. around one curving axis, thereby forming a cylindrical surface, it may be difficult or impossible to prepare an ultrasound transducer having a sufficiently large over-all area and being curved in two directions thereby forming a complex curved surface.

An idea underlying the present invention is to approximate a complex surface of an ultrasound transducer, for example a spheroidal surface, by an approximation using different regions, each comprising a matrix array of ultrasound transducer elements, and combining an over-all ultrasound transducer area from these regions. Therein, at least some of the regions may be curved around specific curving axes, whereas other regions may not be curved, i.e. may be flat, or may only be slightly curved. Particularly, it may be advantageous to provide a flat or only slightly curved region interconnecting adjacent substantially curved regions.

According to a first aspect of the present invention, a cross-shaped ultrasound transducer matrix array according to claim 1 is proposed with a cross-shaped over-all ultrasound transducer area comprising a center region and four branch regions. The center region and the branch regions each comprise a matrix array of ultrasound transducer elements. Therein, the center region has four edges and each of the branch regions extends from one of these edges of the center region. Furthermore, each branch region is curved or bent around an axis parallel to the respective edge of the center region from which the branch region extends. The matrix arrays of each of the center region and the branch regions are two-dimensional matrix arrays comprising transducer elements arranged in a plurality of rows and a plurality of colums.

Accordingly, the over-all ultrasound transducer area may be a combination of the center region and the plurality of branch regions. The over-all ultrasound transducer area may cover a continuous surface with the branch regions directly abutting the center region. In other words, the branch regions may be interconnected via the interposed center region in between.

Each of the matrix arrays included in the center region and the branch regions comprises a plurality of ultrasound transducer elements which are distributed over a 2D area of the respective region. For example, ultrasound transducer elements may be arranged in rows and columns wherein each region comprises a plurality of rows and a plurality of columns.

The over-all ultrasound transducer area comprising the center region and the branch regions forms a cross-shape. Therein, the center may be rectangular with a matrix of A rows and B columns of ultrasound transducer elements. Two branch regions e.g. abutting to the center region at opposing edges may comprise a matrix of A rows and C columns of ultrasound transducer elements whereas two further branch regions abutting to the remaining opposing edges of the center region may comprise a matrix of D rows and B columns of ultrasound transducer elements. In other words, the over-all cross-shaped ultrasound transducer area may comprise four branch regions and an interposed center region wherein the dimensions of the center regions are correlated to the respective widths of the abutting branch regions. Various ratios of A, B, C and D are possible. For example, the center region may be rectangular (A ≠ B) or square (A = B). Furthermore, the branch regions may have identical dimensions (C = D) or may be different (C ≠ D) in their lengths.

Advantageously, the center region is flat. While the branch regions each may be curved around an axis parallel to an abutting edge of the center region and may therefore represent cylindrical surfaces oriented in different directions, it may be preferable to provide the center region without any curvature thereby possibly eliminating the need of a complex compound curvature for the center region interconnecting abutting branch regions. Each of the branch regions may be curved to a same side with respect to a plane including the flat center region.

It may be preferable to arrange at least a portion of the matrix array of transducer elements of the center region and/or of the branch regions with a phased array pitch. Such phased array pitch arrangement may allow for operating the ultrasound transducer in a sector scanning mode in which emitted ultrasound beams may be steered. With such steering, for example, deviations of the center region and/or the branch regions from a cylindrical or spheroidal ideal may be compensated by suitable beam forming using a combination of translation and steering to accommodate the non-cylindrical and/or non-spheroidal geometry.

For example, the matrix array of transducer elements in the center region may be arranged with a phased array pitch in both orthogonal main directions whereas the matrix array of transducer elements of each of the branch regions may be arranged with a phased array pitch only in the main direction parallel to the edge from which the respective branch region extends. In other words, the transducer elements in the center region may have dimensions in both, length and width, of λ/2. The transducer elements in the branch regions may have a dimension of λ/2 in the width direction, i.e. the direction parallel to the edge of the abutting center region, and another dimension such as for example a λ dimension in a length direction perpendicular to the edge of the abutting center region.

While in such an approach, different designs for the transducer element dimensions may be necessary for the center region and the branch regions, the total number of transducer elements and respective control elements controlling each of the transducer elements may be minimized.

Advantageously, the ultrasound elements are arranged on a bendable substrate and each of the ultrasound elements may be provided with an associated control circuit acting as a microbeamformer, which control circuit is preferably integrated in a common substrate. For example, the bendable substrate may be a thin silicon substrate having a thickness which allows for sufficiently bending the substrate together with the ultrasound transducer elements created therewith. Such thickness may be e.g. less than 100 µm, preferably less than 50 µm. In such silicon substrate, the circuitry for controlling a signal to the transducer elements may be integrated.

As noted above, the ultrasound transducer elements may comprise a layer arrangement which may be activated to ultrasonic vibrations. Such layer arrangement may comprise for example one or more piezoelectric layers with respective electrodes. The ultrasound transducer elements furthermore may be provided with a control circuit included in a substrate. Advantageously, the layer arrangement is mechanically attached to the substrate by flip-chip-technology. The application of such flip-chip-technology may provide for connections from each of the array vibration elements to its corresponding circuitry. Such an approach is also referred to as a "flip-chip microbeamformer", where each element's microchannel may be small enough to fit in a space limited by the element's azimuth and elevation dimensions. The microbeamformer may be built in an application specific integrated circuit (ASIC) that may be essentially the same size as the array, optionally plus some extra space around the perimeter for overhead functions. Accordingly, each microchannel may sit behind its element, with electrical contact made through a conductive post between them.

Furthermore, advantageously, the over-all ultrasound transducer area may be composed of a plurality of tiles. For example, one ultrasound transducer matrix array tile may comprise the center region and two of the branch regions arranged at opposite edges of the center region at least one further ultrasound transducer matrix array tile may comprise one or more further branch regions. Alternatively, the over-all ultrasound transducer area may be composed of at least three, preferably four, identical ultrasound transducer matrix array tiles. Each tile may comprise an ASIC comprising specific circuitry for controlling the microbeamformers. Onto the ASIC, an ultrasound transmitting layer may be disposed and electrically connected wherein the respective ultrasound transducer elements may be created by dicing the ultrasound transmitting layer to generate respective elements. Details and possible embodiments using ultrasound transducer matrix array tiles will be described further below with respect to specific embodiments of the present invention.

According to a further aspect of the present invention, a method of fabricating a cross-shaped ultrasound probe is proposed according to claim 12, wherein the method comprises providing a cross-shaped over-all ultrasound transducer area comprising a center region and four branch regions each comprising a matrix array of ultrasound transducer elements wherein each branch region extends from one of four edges of the center region. The method further comprises arranging each branch region in a curved configuration around an axis parallel to the edge of the center region from which the respective branch region extends. The matrix arrays of each of the center region and the branch regions are two-dimensional matrix arrays comprising transducer elements arranged in a plurality of rows and a plurality of colums.

The over-all ultrasound transducer area may be composed from a plurality of tiles. Each tile may comprise an ASIC in which a control circuitry may be prepared with standard semiconductor technologies. On a surface of the ASIC, a layer may be deposited or bonded which may be made from a material which can be activated for ultrasound vibration. This layer may then be diced to prepare a multiplicity of transducer elements arranged in matrix arrays wherein each elements may be controlled by its associated ASIC. In the branch regions, at least some of the dicing cuts may be arranged parallel to the edge of the center region from which the respective branch region extends. Thereby, assuming a flexible substrate for the ASICs, the branch regions may be bent into a shape curved around an axis parallel to the respective edges. It has to be noted that aspects and embodiments of the present invention are described herein with reference to different subject-matters. In particular, some embodiments are described with respect to the ultrasound probe whereas other embodiments are described with respect to the method of fabricating the ultrasound probe. However, a person skilled in the art will gather from the above and the following description that, unless other notified, in addition to any combination of features belonging to one type of subject-matter also any combination between features relating to different subject-matters, in particular between features of the apparatus type claims and features of the method type claims, is considered to be disclosed with this application.

### BRIEF DESCRIPTION OF THE DRAWINGS

Features and advantages of the present invention will be further described with respect to specific embodiments as shown in the accompanying figures but to which the invention shall not be limited.
Fig. 1 shows a conventional curved matrix array of ultrasound transducer elements.
Fig. 2a,b,c,d show representations of a field of view of the matrix array of Fig. 1 in perspective view, top view, azimuth view and elevation view, respectively.
Fig. 3 shows a perspective view of a matrix array arrangement for an ultrasound probe according to an embodiment of the present invention.
Fig. 4 shows a perspective view of a matrix array arrangement for an ultrasound probe with a flat center region according to another embodiment of the present invention.
Fig. 5 shows a cross-section of a matrix array arrangement for the ultrasound probe of Fig. 4.
Figs. 6a,b,c show representations of a field of view of an ultrasound probe with a linear array pitch according to an embodiment of the present invention in perspective view, top view and azimuth/elevation view, respectively.
Figs. 7a,b,c show representations of a field of view of an ultrasound probe with a phased array pitch according to an embodiment of the present invention in perspective view, top view and azimuth/elevation view, respectively.
Fig. 8 shows a cross sectional view of a matrix array of ultrasound transducers for use in an embodiment of the present invention.
Fig. 9 shows an ultrasound probe arrangement composed of three tiles according to an embodiment of the present invention.
Fig. 10 shows an ultrasound probe arrangement composed of four tiles according to an embodiment of the present invention.
Fig. 11 shows an ultrasound probe arrangement comprising four identical tiles according to an embodiment of the present invention.
Fig 12 shows a top view of a matrix array arrangement for an ultrasound probe arrangement according to an embodiment of the present invention prior to curving.
Fig. 13 shows a top view of a matrix array arrangement for an ultrasound probe with a matrix array arrangement optimized for minimizing the number of transducer elements according to an embodiment of the present invention.

Features shown in the figures are only schematical and not to scale. Same or similar features are labeled with same or similar reference signs. Furthermore, lines on surfaces shown in the figures do not necessarily indicate any actual connections or even segments of transducer elements but may refer to surface contours intended to help make the 3D surface more apparent in a 2D drawing.

### DETAILED DESCRIPTION OF THE INVENTION

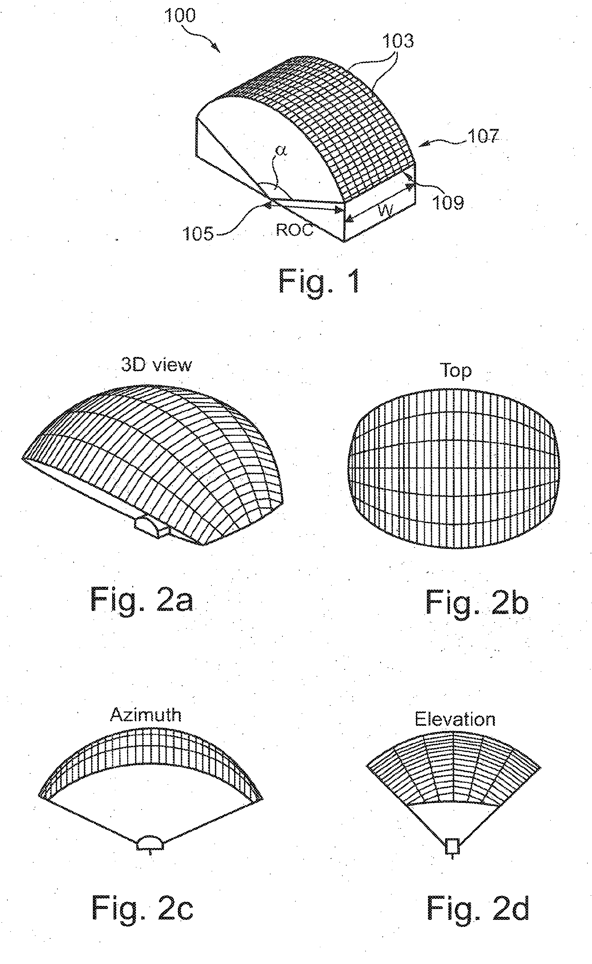

Fig. 1 shows a conventional matrix array 100 of ultrasound transducer elements 103 arranged along a surface which is bent around an axis 105. The matrix array comprises a plurality of columns 107 and rows 109 of ultrasound transducer elements 103 arranged on a two-dimensional surface. The exemplary curved matrix array has a radius of curvature ROC of 11.5 mm, a field of view angle a of 130° in azimuth, and an aperture width w in elevation of 10 mm. The ultrasound transducer elements 103 are arranged with a phased-array pitch in elevation direction to support 90° steering.

Figs. 2a,b,c and d show a perspective view, a top view, an azimuth view and an elevation view of the field of view obtainable with the curved matrix array shown in Fig. 1.

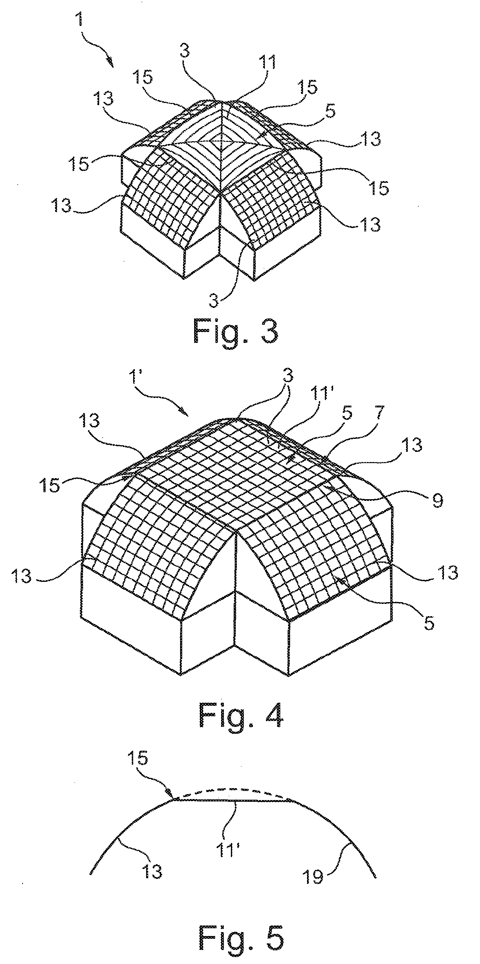

Fig. 3 shows a perspective view of an ultrasound transducer matrix array 1 for an ultrasound probe, the matrix array 1 having a curved cross-shaped form and comprising a center region 11 interconnecting four branch regions 13. Each of the regions 11, 13 comprises a matrix array 5 of ultrasound transducers 3. The branch regions 13 extend from respective edges 15 of the center region 11. Each of the branch regions 13 is curved around an axis parallel to the respective edge 15 from which it extends.

In the embodiment shown in Fig. 3, the center region 11 may have a complex curved surface which is curved in two directions. However, it may be difficult to fabricate a matrix array of transducer elements with such complex curvature. This may be due for example to the fact that the material from which the transducer elements are made may be bendable around one axis thereby being easily curved in a cylindrical surface but may not be deformed in a complex curved shape due to a lacking mechanical flexibility/elasticity.

Accordingly, it may be advantageous to provide an ultrasound transducer matrix array 1' with a flat center region 11' as shown in Fig. 4. The flat center region 11' may have a rectangular shape with four edges 15 from which curved branch regions 13 extend. The matrix array 5 of the flat center region 11' may comprise A columns 7 and B rows 9 of ultrasound transducer elements 3. In the embodiment of Fig. 4, the center region 11' is square (A = B). The matrix arrays 5 of the branch regions 13 directly abut to one of the edges 15 of the center region 11' and each have the same width as the dimension of the edge 15 of the center region 11' from which they extend. Accorddingly, the branch regions 13 may have A or B transducer elements 3 in a width direction. However, their length may be different. In principle, each of the branch regions may have a different length and/or a different radius of curvature; however, it may be advantageous to provide each of the branch regions with a same length and a same radius of curvature thereby achieving a symmetric arrangement.

Fig. 5 shows a cross-section of the ultrasound transducer matrix array 1' shown in Fig. 4. Two curved branch regions 13 extend from the flat center region 11' at edges 15.

The field of view of ultrasound probe with a cross-shaped curved ultrasound transducer matrix array as shown in Fig. 4 may not be a complete spheroid; there may be gaps in the "corners" where neither of the orthogonal apertures can provide adequate image quality.

The actual field of view of such cross-shaped ultrasound transducer may depend on both the curved surface provided by the basic geometry of the matrix arrays of ultrasound transducer elements, plus the available steering which may be dependent on the element pitch.

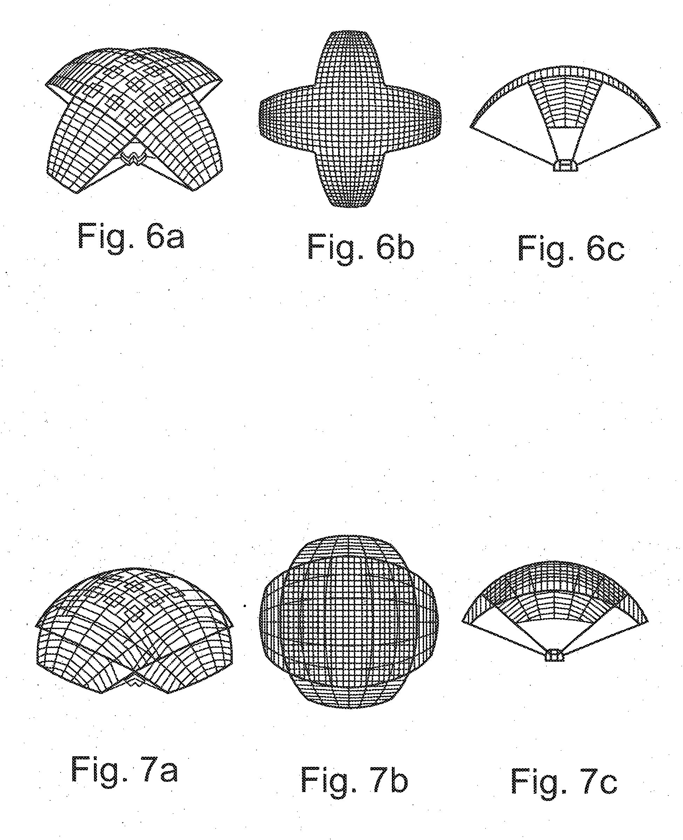

If the entire matrix array is diced on a linear array pitch in both azimuth and elevation, steering is usually limited to about 45°. Figs. 6a,b,c illustrate the resulting field of view in a perspective view, top view and azimuth/elevation view, respectively. This approach may provide coverage of about half of an ideal spheroidal field of view, with gaps in the "corners" which may be beyond the combined translation and steering of the composite array.

Such a transducer may also provide improved image quality in the transverse plane through the center of the array, over a larger field of view, than would be possible with a single curved matrix array as for example shown in Fig. 1. In addition to the field of view improvements as noted above the image quality in the transverse plane may improve as well by providing for curved linear array operation in that direction, instead of relying only on steering. The curved matrix array shown in Fig. 1 may achieve a 90° field of view in elevation as shown in Fig. 2d only if it has phased-array pitch in elevation, whereas the cross-shaped array shown in Figs. 3 or 4 has a 130° field of view in elevation even if diced at a larger linear-array pitch. The improved image quality results from the larger available aperture in the elevation direction.

It may be noted that such cross-shaped array as shown in Figs. 3 or 4 may have fewer ultrasound transducer elements and respective microchannels than a conventional curved matrix array as shown in Fig. 1. For example, let the conventional curved matrix array shown in Fig. 1 comprise 128 columns and 64 rows; therefore, it comprises 128 x 32 = 8192 individual transducer elements and microchannels. To obtain a 90° field of view, the B rows must be diced on a phased array pitch. Let the cross-shaped array shown in Figs. 3 or 4 be symmetrical, where the dimensions of either major axis be the same as the array in Fig. 1. Furthermore, let the array be diced on linear array pitch in both directions; there will be 128 columns in the azimuth direction and 32 rows in the elevation direction for each of the crossed arrays. The total number of elements in the array will then be 2 x (128 x 32) - 32 x 32 = 7168 individual array elements. Although the crossed array may have more surface area, it has fewer array elements because it is diced at linear array pitch in both directions.

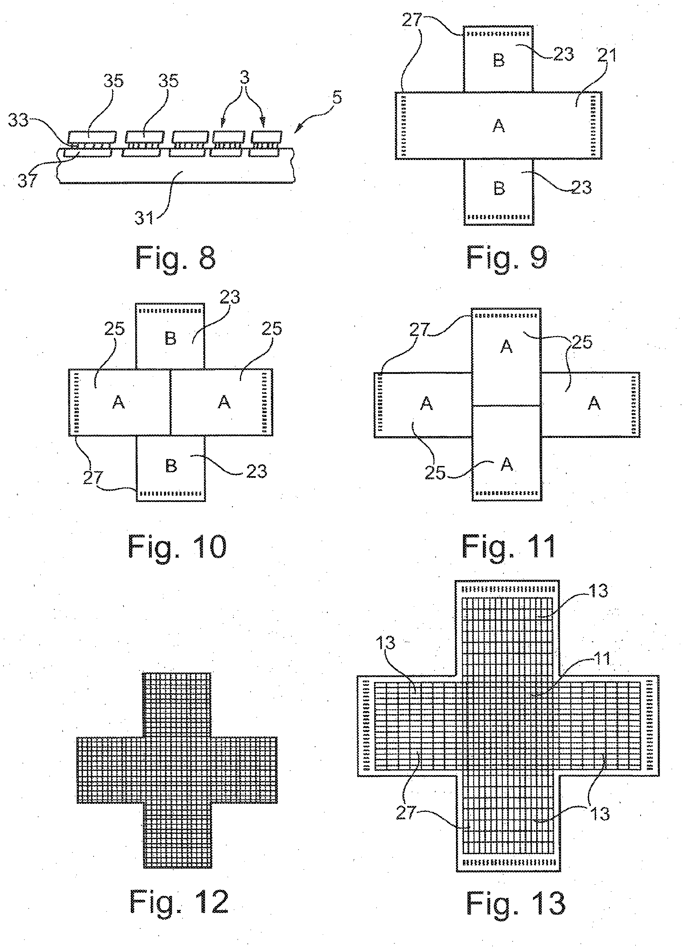

Figs. 7a,b,c show a perspective view, a top view and an azimuth/elevation view, respectively, of a field of view for an ultrasound probe with an ultrasound transducer matrix array according to an embodiment of the present invention in which the entire array is diced to provide phased array pitch in elevation. This may provide about 90° of steering. In this case, the increased steering may provide for much more coverage of an ideal spheroidal field of view. As will be further described below with respect to Fig. 9, it may not be necessary to dice the entire array at phased array pitch.

In the embodiment shown in Fig. 7, matrix arrays having a 130° azimuth field of view as discussed further above with respect to the conventional curved matrix array show in Fig. 1 have been used for the various regions of the ultrasound probe. The result may be an improvement in field of view of somewhat less than 50% over the conventional single curved matrix array of Fig. 1 with 90° steering. If the azimuth field of view is increased above 130°, then the field of view improvement may increase beyond what is illustrated in Fig. 7.

In addition to the field of view improvements as noted above the image quality in the transverse plane may improve as well by providing for curved linear array operation in that direction, instead of relying only on steering.

Fig. 8 shows a cross sectional view of a matrix array 5 of ultrasound transducers 3. In a common bendable substrate 31, a plurality control circuits 37 have been integrated. Each of the control cirsuits 37 is connected via connections 33 to an associated layer arrangement 35 which may be activated to ultrasonic vibrations. The layer arrangement may comprise a piezoelectric layer together with one or more additional layers for improving acoustic impedance match, thereby forming an acoustic stack. The connections 33 have been established via flip-chip technology. The plurality of vibration layer arrangements 35 may be produced by first connecting an over-all layer arrangement to the substrate 31 and subsequently dicing it thereby separating the plurality of vibration layers arrangement 35.

As shown in Figs. 9 to 11, an over-all ultrasound transducer area of an ultrasound probe according to embodiments of the present invention may be composed of a plurality of ultrasound transducer matrix array tiles 21, 23, 25. The over-all ultrasound transducer area may comprise at least three, but probably four or more, ASICs. In the embodiments shown in Figs. 9 to 11, a symmetrical over-all ultrasound transducer area is shown, but symmetry is not a requirement.

Figs. 9 and 10 illustrate two embodiments in which two different ASIC designs A, B are used. The two designs may be most likely very similar and the system I/O 27 may be on the outer edges of the ASICs. The ASICs may be tiled prior to bonding to the acoustic stack. Alternatively, pre-bonded ASIC/acoustic stack sub-assemblies may be tiled. The pre-bonded sub-assemblies may be diced prior to tiling if either
(i) the tiling alignment is good enough, or
(ii) there is a calibration procedure for dealing with any misregistrations.

If not, then the over-all transducer area may be diced after tiling as described below. The tiling should be done on a substrate that either is or may be made flexible for later curving.

If the ASICs are tiled before bonding to the acoustic stack, then there may be two basic choices for probe configuration. The tiled ASIC sub-assembly may be bonded to a single large acoustic stack, but this might result in a significant amount of waste from unused corners (4/9 of the over-all area for a design with square symmetry). If the process allows use of multiple probe sub-assemblies, then the probe may comprise either three or four pieces, in the same configuration as the ASIC as illustrated in Figs. 9 and 10.

It is also possible to use the same approach with a single ASIC design, as shown in Fig. 11. In this case, the two orthogonal apertures may not be the same.

At this point, the ultrasound unit including the over-all transducer area may be ready for dicing, assuming that the ASIC/probe sub-assemblies are not already diced when tiled. When diced after tiling, the array elements may be properly registered with respect to each other, although the underlying ASICs may be slightly misaligned.

Fig. 12 shows an array geometry after dicing. The unit may then be ready for curving. The substrate may be made sufficiently bendable by using a bendable material. Alternatively, a rigid material may be made bendable by back-dicing. The tools and techniques for getting the suitable radius of curvature may be similar to those used for conventional one-dimensional transducer arrays. For one-dimensional transducer arrays, one approach may be to place the array face down into a curved tool that provides a specified radius of curvature, pushed into contact with the face of the tool, and bonded to a mechanical support structure behind the ASIC/array sub-assembly. Another approach may be to bond the array face up over a mechanical support structure. Either of these methods may be used for the conventional curved matrix transducer array shown in Fig. 1. Similar techniques may be used for the cross-shaped matrix arrays, except that the tool and mechanical support may be more complex because there may be a flat center region in the middle and there may be two sets of curved branch regions extending therefrom.

After curving, array manufacture may be completed using techniques similar to those used on conventional matrix arrays for ultrasound transducer probes.

As noted above, it may not be necessary to dice the entire array at phased array pitch to achieve the field of view illustrated in Fig. 7. Fig. 13 illustrates a dicing pattern that provides for 90° steering in the elevation direction for each orthogonal sub-set of the over-all array. While dicing in each direction, the pitch may be halved the center region. This may result in elements that are λ in azimuth and λ/2 in elevation in the branch regions 13, and λ/2 x λ/2 in the flat center region 11. This approach may need different microchannel designs for each of the matrix arrays forming the center region 11 and the branch regions 13, but it may minimize the total number of microchannels.

In order to accommodate the dicing approach of Fig. 13 to the specific tiling arrangement of Fig. 11, it may be necessary to incorporate sufficient flexibility for some microchannel locations on the chip of an ASIC design to deal with either λ x λ/2 or λ/2 x λ/2 elements.

It should be noted that the terms such as "comprising" do not exclude other elements or steps and that the indefinite article "a" or "an" does not exclude a plurality of elements. Also elements described in association with different embodiments may be combined. It should also be noted that reference signs in the claims shall not be construed as limiting the scope of the claims.

## Claims

1. A cross-shaped ultrasound transducer matrix array (1) for an ultrasound probe, comprising a cross-shaped over-all ultrasound transducer area comprising:
a center region (11) comprising a matrix array (5) of ultrasound transducers elements (3), wherein the center region (11) has four edges (15), and
four branch regions (13) each comprising a matrix array (5) of ultrasound transducers elements (3), wherein each branch region (13) extends from one of the edges (15) of the center region (11) and wherein each branch region (13) is curved around an axis parallel to the edge (15) of the center region (11) from which the respective branch region (13) extends,
**characterized in that** the matrix arrays (5) of each of the center region (11) and the branch regions (13) are two-dimensional matrix arrays comprising transducer elements (3) arranged in a plurality of rows and a plurality of columns.

2. The ultrasound transducer matrix array of claim 1, wherein the center region (11') is flat.

3. The ultrasound transducer matrix array of claim 1 or 2, comprising a rectangular center region (11) with a matrix of A rows and B columns of ultrasound transducer elements (3) and two branch regions (13) each with a matrix of A rows and C columns of ultrasound transducer elements (3) and two branch regions (13) each with a matrix of D rows and B columns of ultrasound transducer elements (3).

4. The ultrasound transducer matrix array of one of claims 1 to 3, wherein at least a portion of the matrix array of transducer elements (3) of the center region (11) and/or of the branch regions (13) is arranged with a phased array pitch.

5. The ultrasound transducer matrix array of one of claims 1 to 4, wherein the matrix array of transducer elements (3) of the center region (11) is arranged with a phased array pitch in both main directions and wherein the matrix array of transducer elements of each of the branch regions (13) is arranged with a phased array pitch only in the main directions parallel to the edge (15) from which the respective branch region (13) extends.

6. The ultrasound transducer matrix array of one of claims 1 to 5, wherein ultrasound transducer elements (3) is provided with a bendable substrate (31).

7. The ultrasound transducer matrix array of one of claims 1 to 6, wherein each of the ultrasound transducer elements (3) has an associated control circuit (37).

8. The ultrasound transducer matrix array of claim 7, wherein associated control circuits (37) are integrated in a common substrate (31).

9. The ultrasound transducer matrix array of claim 8, wherein each of the ultrasound transducer elements (3) comprise a layer arrangement (35) which may be activated to ultrasonic vibrations and is provided with a control circuit (37) included in a substrate (31) wherein the layer arrangement (35) is is mechanically attached to the substrate (31) by flip-chip-technology.

10. The ultrasound transducer matrix array of one of claims 1 to 9, wherein the over-all ultrasound transducer area is composed of
one ultrasound transducer matrix array tile (21) comprising the center region (11) and two of the branch regions (13) arranged at opposite edges of the center region (11), and
at least one ultrasound transducer matrix array tile (23) comprising a further branch region (13).

11. The ultrasound transducer matrix array of one of claims 1 to 9, wherein the over-all ultrasound transducer area is composed of at least three identical ultrasound transducer matrix array tiles (25).

12. A method of fabricating a cross-shaped ultrasound transducer matrix array for an ultrasound probe (1), comprising:
- providing a cross-shaped over-all ultrasound transducer area comprising
a center region (11) comprising a matrix array (5) of ultrasound transducer elements (3), wherein the center region (11) has four edges (15), and
four branch regions (13) each comprising a matrix array (5) of ultrasound transducer elements (3), wherein each branch region (13) extends from one of the edges (15) of the center region (11);
- arranging each branch region (13) in a curved configuration around an axis parallel to the edge (15) of the center region (11) from which the respective branch region (13) extends
**characterized in that** the matrix arrays (5) of each of the center region (11) and the branch regions (13) are two-dimensional matrix arrays comprising transducer elements (3) arranged in a plurality of rows and a plurality of columns.

13. The method of claim 12, wherein the center region is arranged in a flat configuration.

14. The method of claim 12 or 13, wherein each of the ultrasound transducer elements (3) comprise a layer arrangement (35) which may be activated to ultrasonic vibrations and is provided with a control circuit (37) included in a substrate (31), wherein the layer arrangement (35) is is mechanically attached to the substrate (31) by flip-chip-technology.

## Patentansprüche

1. Kreuzförmiges Ultraschallwandler-Matrixarray (1) für eine Ultraschallsonde, umfassend eine kreuzförmige Gesamtultraschallwandlerfläche, die Folgendes umfasst:
eine Mittenregion (11) umfassend ein Matrixarray (5) von Ultraschallwandlerelementen (3), wobei die Mittenregion (11) vier Ränder (15) hat, und
vier Zweigregionen (13), die jeweils ein Matrixarray (5) von Ultraschallwandlerelementen (3) umfassen, wobei sich jede Zweigregion (13) von einem der Ränder (15) der Mittenregion (11) aus erstreckt und wobei jede Zweigregion (13) um eine Achse herum gekrümmt ist, die parallel zum Rand (15) der Mittenregion (11) verläuft, von der sich die betreffende Zweigregion (13) aus erstreckt,
**dadurch gekennzeichnet, dass** die Matrixarrays (5) von sowohl der Mittenregion (11) als auch der Zweigregionen (13) zweidimensionale Matrixarrays sind, die Wandlerelemente (3) umfassen, welche in einer Vielzahl von Reihen und einer Vielzahl von Spalten angeordnet sind.

2. Ultraschallwandler-Matrixarray nach Anspruch 1, wobei die Mittenregion (11') flach ist.

3. Ultraschallwandler-Matrixarray nach Anspruch 1 oder 2, umfassend eine rechteckige Mittenregion (11) mit einer Matrix aus A Reihen und B Spalten von Ultraschallwandlerelementen (3) und zwei Zweigregionen (13) jeweils mit einer Matrix aus A Reihen und C Spalten von Ultraschallwandlerelementen (3) und zwei Zweigregionen (13) jeweils mit einer Matrix aus D Reihen und B Spalten von Ultraschallwandlerelementen (3).

4. Ultraschallwandler-Matrixarray nach einem der Ansprüche 1 bis 3, wobei mindestens ein Teil des Matrixarrays aus Wandlerelementen (3) der Mittenregion (11) und/oder der Zweigregionen (13) mit einem Phased-Array-Abstand angeordnet ist.

5. Ultraschallwandler-Matrixarray nach einem der Ansprüche 1 bis 4, wobei das Matrixarray aus Wandlerelementen (3) der Mittenregion (11) mit einem Phased-Array-Abstand in beiden Hauptrichtungen angeordnet ist und wobei das Matrixarray von Wandlerelementen von jeder der Zweigregionen (13) mit einem Phased-Array-Abstand nur in den Hauptrichtungen parallel zu dem Rand (15) angeordnet ist, von dem aus sich die betreffende Zweigregion (13) erstreckt.

6. Ultraschallwandler-Matrixarray nach einem der Ansprüche 1 bis 5, wobei Ultraschallwandlerelemente (3) mit einem biegbaren Substrat (31) versehen sind.

7. Ultraschallwandler-Matrixarray nach einem der Ansprüche 1 bis 6, wobei jedes der Ultraschallwandlerelemente (3) eine zugehörige Steuerschaltung (37) hat.

8. Ultraschallwandler-Matrixarray nach Anspruch 7, wobei die zugehörigen Steuerschaltungen (37) in einem gemeinsamen Substrat (31) integriert sind.

9. Ultraschallwandler-Matrixarray nach Anspruch 8, wobei jedes der Ultraschallwandlerelemente (3) eine Schichtanordnung (35) umfasst, die für Ultraschallschwingungen aktiviert werden kann und mit einer in einem Substrat (31) enthaltenen Steuerschaltung (37) versehen ist, wobei die Schichtanordnung (35) mechanisch durch Flip-Chip-Technologie an dem Substrat (31) angebracht ist.

10. Ultraschallwandler-Matrixarray nach einem der Ansprüche 1 bis 9, wobei die Gesamtultraschallwandlerfläche zusammengesetzt ist aus
einer Ultraschallwandler-Matrixarraykachel (21) umfassend eine Mittenregion (11) und zwei der Zweigregionen (13), die an gegenüberliegenden Rändern der Mittenregion (11) angeordnet sind, und
mindestens einer Ultraschallwandler-Matrixarraykachel (23) umfassend eine weitere Zweigregion (13).

11. Ultraschallwandler-Matrixarray nach einem der Ansprüche 1 bis 9, wobei die Gesamtultraschallwandlerfläche zusammengesetzt ist aus mindestens drei identischen Ultraschallwandler-Matrixarraykacheln (25).

12. Verfahren zum Herstellen eines kreuzförmigen Ultraschallwandler-Matrixarrays für eine Ultraschallsonde (1), wobei das Verfahren Folgendes umfasst:
- Bereitstellen einer kreuzförmigen Gesamtultraschallwandlerfläche, die Folgendes umfasst:
eine Mittenregion (11) umfassend ein Matrixarray (5) von Ultraschallwandlerelementen (3), wobei die Mittenregion (11) vier Ränder (15) hat, und
vier Zweigregionen (13), die jeweils ein Matrixarray (5) von Ultraschallwandlerelementen (3) umfassen, wobei sich jede Zweigregion (13) von einem der Ränder (15) der Mittenregion (11) aus erstreckt;
- Anordnen jeder Zweigregion (13) in einer gekrümmten Konfiguration um eine Achse herum, die parallel zum Rand (15) der Mittenregion (11) verläuft, von der sich die betreffende Zweigregion (13) aus erstreckt,
**dadurch gekennzeichnet, dass** die Matrixarrays (5) von sowohl der Mittenregion (11) als auch der Zweigregionen (13) zweidimensionale Matrixarrays sind, die Wandlerelemente (3) umfassen, welche in einer Vielzahl von Reihen und einer Vielzahl von Spalten angeordnet sind.

13. Verfahren nach Anspruch 12, wobei die Mittenregion in einer flachen Konfiguration angeordnet ist.

14. Verfahren nach Anspruch 12 oder 13, wobei jedes der Ultraschallwandlerelemente (3) eine Schichtanordnung (35) umfasst, die für Ultraschallschwingungen aktiviert werden kann und mit einer in einem Substrat (31) enthaltenen Steuerschaltung (37) versehen ist, wobei die Schichtanordnung (35) mechanisch durch Flip-Chip-Technologie an dem Substrat (31) angebracht ist.

## Revendications

1. Réseau matriciel de transducteurs ultrasonores cruciforme (1) pour une sonde ultrasonore, comprenant une zone de transducteurs ultrasonores globale cruciforme comprenant :
une région centrale (11) comprenant un réseau matriciel (5) d'éléments de transducteurs ultrasonores (3), la région centrale (11) possédant quatre bords (15), et
quatre régions formant branches (13), chacune comprenant un réseau matriciel (5) d'éléments de transducteurs ultrasonores (3), chaque région formant branche (13) s'étendant depuis l'un des bords (15) de la région centrale (11) et chaque région formant branche (13) étant incurvée autour d'un axe parallèle au bord (15) de la région centrale (11) à partir duquel s'étend la région formant branche respective,
**caractérisé en ce que** les réseaux matriciels (5) de chacune de la région centrale (11) et des régions formant branches (13) sont des réseaux matriciels bidimensionnels comprenant des éléments formant transducteurs (3) agencés en une pluralité de rangées et une pluralité de colonnes.

2. Réseau matriciel de transducteurs ultrasonores selon la revendication 1, dans lequel la région centrale (11') est plane.

3. Réseau matriciel de transducteurs ultrasonores selon la revendication 1 ou 2, comprenant une région centrale rectangulaire (11) avec une matrice de rangées A et de colonnes B d'éléments formant transducteurs ultrasonores (3) et deux régions formant branches (13), chacune avec une matrice de rangées A et de colonnes C d'éléments formant transducteurs ultrasonores (3) et deux régions formant branches (13), chacune avec une matrice de rangées D et de colonnes B d'éléments formant transducteurs ultrasonores (3).

4. Réseau matriciel de transducteurs ultrasonores selon l'une quelconque des revendications 1 à 3, dans lequel au moins une partie du réseau matriciel d'éléments formant transducteurs (3) de la région centrale (11) et/ou des régions formant branches (13) est agencée avec un pas de réseau à commande de phase.

5. Réseau matriciel de transducteurs ultrasonores selon l'une quelconque des revendications 1 à 4, dans lequel le réseau matriciel d'éléments formant transducteurs (3) de la région centrale (11) est agencé avec un pas de réseau à commande de phase dans les deux directions principales et dans lequel le réseau matriciel des éléments formant transducteurs de chacune des régions formant branches (13) n'est agencé avec un pas de réseau à commande de phase que dans les directions principales parallèles au bord (15) à partir duquel s'étend la région formant branche (13) respective.

6. Réseau matriciel de transducteurs ultrasonores selon l'une quelconque des revendications 1 à 5, dans lequel les éléments formant transducteurs ultrasonores (3) sont dotés d'un substrat flexible (31).

7. Réseau matriciel de transducteurs ultrasonores selon l'une quelconque des revendications 1 à 6, dans lequel chacun des éléments formant transducteurs ultrasonores (3) a un circuit de commande associé (37).

8. Réseau matriciel de transducteurs ultrasonores selon la revendication 7, dans lequel les circuits de commande associés (37) sont intégrés dans un substrat commun (31).

9. Réseau matriciel de transducteurs ultrasonores selon la revendication 8, dans lequel chacun des éléments formant transducteurs ultrasonores (3) comprend une structure de couches (35) qui peut être activée par des vibrations ultrasonores et est doté d'un circuit de commande (37) inclus dans un substrat (31) dans lequel la structure de couches (35) est fixée mécaniquement au substrat (31) à l'aide de la technologie de connexion par billes.

10. Réseau matriciel de transducteurs ultrasonores selon l'une quelconque des revendications 1 à 9, dans lequel la zone de transducteurs ultrasonores globale est composée
d'une mosaïque de réseaux matriciels de transducteurs ultrasonores (21) comprenant la région centrale (11) et deux des régions formant branches (13) placées au niveau de bords opposés de la région centrale (11), et
au moins une mosaïque de réseaux matriciels de transducteurs ultrasonores (23) comprenant une région formant branche supplémentaire (13).

11. Réseau matriciel de transducteurs ultrasonores selon l'une des revendications 1 à 9, dans lequel la zone de transducteurs ultrasonores globale est composée d'au moins trois mosaïques de réseau matriciel de transducteurs ultrasonores (25).

12. Procédé de fabrication d'un réseau matriciel de transducteurs ultrasonores cruciforme pour une sonde ultrasonore (1), comprenant
- la fourniture d'une zone de transducteurs ultrasonores globale cruciforme comprenant :
une région centrale (11) comprenant un réseau matriciel (5) d'éléments formant transducteurs ultrasonores (3), dans lequel la région centrale (11) a quatre bords (15), et
quatre régions formant branches (13), chacune comprenant un réseau matriciel (5) d'éléments formant transducteurs ultrasonores (3), dans lequel chaque région formant branche (13) s'étend depuis l'un des bords (15) de la région centrale (11) ;
- l'agencement de chaque région formant branche (13) dans une configuration incurvée autour d'un axe parallèle au bord (15) de la région centrale (11) à partir duquel s'étend la région formant branche respective (13)
**caractérisé en ce que** les réseaux matriciels (5) de chacune de la région centrale (11) et des régions formant branche (13) sont des réseaux matriciels bidimensionnels comprenant des éléments formant transducteurs (3) agencés en une pluralité de rangés et une pluralité de colonnes.

13. Procédé selon la revendication 12, dans lequel la région centrale est agencée dans une configuration plane.

14. Procédé selon la revendication 12 ou 13, dans lequel chacun des éléments formant transducteurs ultrasonores (3) comprend une structure de couches (35) qui peut être activée par des vibrations ultrasonores et est doté d'un circuit de commande (37) inclus dans un substrat (31), dans lequel la structure de couches (35) est fixée mécaniquement au substrat (31) à l'aide de la technologie de connexion par billes.
